(19) Europäisches Patentamt
European Patent Office
Office européen des brevets

(11) EP 3 019 457 B1

(12) **EUROPÄISCHE PATENTSCHRIFT**

(45) Veröffentlichungstag und Bekanntmachung des Hinweises auf die Patenterteilung:
06.06.2018 Patentblatt 2018/23

(21) Anmeldenummer: 14732569.0

(22) Anmeldetag: 25.06.2014

(51) Int Cl.:
C07C 41/06 (2006.01)     C07C 2/08 (2006.01)
C07C 45/50 (2006.01)     C07C 4/02 (2006.01)
C07C 5/03 (2006.01)     C07C 5/48 (2006.01)
C07D 305/10 (2006.01)     C07C 2/10 (2006.01)
C07C 29/04 (2006.01)     C10G 50/00 (2006.01)

(86) Internationale Anmeldenummer:
PCT/EP2014/063374

(87) Internationale Veröffentlichungsnummer:
WO 2014/207034 (31.12.2014 Gazette 2014/53)

(54) **OLIGOMERIZATOIN VON C4-STRÖMEN MIT MINIMALEM GEHALT AN 1-BUTEN**

OLIGOMERIZATOIN OF C4 FLOWS HAVING MINIMAL 1-BUTENE CONTENT

OLIGOMERIZATOIN DE FLUX C4 À FAIBLE TENEUR EN 1-BUTÈNE

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(30) Priorität: 27.06.2013 DE 102013212481

(43) Veröffentlichungstag der Anmeldung:
18.05.2016 Patentblatt 2016/20

(73) Patentinhaber: Evonik Degussa GmbH
45128 Essen (DE)

(72) Erfinder:
• PEITZ, Stephan
  45739 Oer-Erkenschwick (DE)
• GEILEN, Frank
  45721 Haltern am See (DE)
• MASCHMEYER, Dietrich
  45657 Recklinghausen (DE)
• STOCHNIOL, Guido
  45721 Haltern am See (DE)

(56) Entgegenhaltungen:
WO-A1-99/25668

**Beschreibung**

Die vorliegende Erfindung betrifft ein Verfahren nach dem Oberbegriff des Anspruch 1. Ein solches Verfahren ist als "OCTOL-Prozess" bekannt.

Unter der Oligomerisierung versteht man die Reaktion von Kohlenwasserstoffen mit sich selbst, wobei entsprechend längerkettige Kohlenwasserstoffe entstehen. Oligomerisieren lassen sich insbesondere Olefine (= Alkene), dies sind aliphatische Kohlenwasserstoffe, die mindestens eine Kohlenstoff-Doppelbindung im Molekül besitzen. So lässt sich beispielsweise durch die Oligomerisierung von zwei Olefinen mit drei Kohlenstoffatomen (Dimerisierung) ein Olefin mit sechs Kohlenstoffatomen aufbauen. Verbinden sich hingegen drei Olefine mit drei Kohlenstoffatomen miteinander (Trimerisierung), entsteht ein Olefin mit neun Kohlenstoffatomen. Werden Butene - das sind Olefine mit vier Kohlenstoffatomen - einer Oligomerisierung unterworfen, entstehen dabei im Wesentlichen Olefine mit acht Kohlenstoffatomen (im Folgenden $C_8$-Olefine, sonst auch oft "Dibutene" genannt), Olefine mit zwölf Kohlenstoffatomen ($C_{12}$-Olefine, "Tributene") sowie in einem geringeren Umfang Olefine mit mehr als zwölf Kohlenstoffatomen ($C_{12+}$-Olefine).

Die Erfindung befasst sich ausschließlich mit der Oligomerisierung von $C_4$-Olefinen, bei der überwiegend durch Dimerisierung und Trimerisierung $C_8$- und $C_{12}$-Olefine entstehen.

Zu den $C_4$- Olefinen gehören die vier isomeren Stoffe 1-Buten, cis-2-Buten, trans-2-Buten und Isobuten. 1-Buten und die beiden 2-Butene gehören dabei zur Gruppe der linearen Butene, während Isobuten ein verzweigtes Olefin darstellt. Die linearen $C_4$-Olefine 1-Buten, cis-2-Buten und trans-2-Buten werden auch als "n-Butene" zusammengefasst.

Einen aktuellen Überblick über die chemischen und physikalischen Eigenschaften der Butene sowie deren technische Aufarbeitung und Verwertung bieten:

- F. Geilen, G. Stochniol, S. Peitz and E. Schulte-Koerne: Butenes. Ullmann's Encyclopedia of Industrial Chemistry. (2013)

Butene fallen beim Cracken von Erdölfraktionen in einem Steamcracker oder in einem fluidkatalytischen Cracker (FCC) an. Allerdings werden die Butene dabei nicht rein erhalten sondern als sogenannter "$C_4$-Schnitt". Dies ist ein je nach Herkunft unterschiedlich zusammengesetztes Gemisch aus Kohlenwasserstoffen mit vier Kohlenstoffatomen, welches neben $C_4$-Olefinen auch gesättigte $C_4$-Kohlenwasserstoffe (Alkane) enthält. Des Weiteren können Spuren von Kohlenwasserstoffen mit mehr oder weniger als vier Kohlenstoffatomen (beispielsweise aber nicht ausschließlich Propan und/oder Pentene) und anderen organischen oder anorganischen Begleitstoffen enthalten sein. Eine alternative Quelle für Butene sind beispielsweise chemische Prozesse, wie die Dehydrierung von Butanen, sowie die fermentative oder die pyrolytische Umwandlung von nachwachsenden Rohstoffen.

Alkane sind Kohlenwasserstoffe, bei denen die Kohlenstoffatome ausschließlich über Einfachbindungen miteinander verknüpft sind und die aufgrund dieser Einfachbindung deutlich weniger reaktiv sind als die entsprechenden Olefine. Alkane werden daher im Gegensatz zu den Olefinen kaum als Ausgangsstoff für chemische Reaktionen genutzt sondern dienen meist als Brennstoff oder Treibmittel. Zu den $C_4$-Alkanen zählen n-Butan und Isobutan. Die Butane sind in den meisten industriell verfügbaren $C_4$-Schnitten enthalten und verhalten sich in der Oligomerisierung inert.

Die Herstellung von $C_8$-Olefin und von $C_{12}$-Olefin aus den in $C_4$-Schnitten enthaltenen Butenen ist wirtschaftlich attraktiv und wird deswegen großindustriell betrieben. Die erhaltenen $C_8$-Olefine lassen sich nämlich im Wege der Hydroformylierung mit Kohlenmonoxid und Wasserstoff zu $C_9$-Aldehyden umsetzen, die durch anschließende Hydrierung zu $C_9$-Alkoholen weiterverarbeitet werden. Die $C_9$-Alkohole stellen wiederum einen begehrten Ausgangsstoff für die Herstellung von Weichmachern für PVC dar. In analoger Weise werden die bei der Oligomerisierung von drei Butenen entstehenden $C_{12}$-Olefine durch Hydroformylierung und Hydrierung zu $C_{13}$-Alkoholen weiterverarbeitet. Die $C_{13}$-Alkohole sind ein Vorprodukt für die Herstellung waschaktiver Substanzen. Da die Nachfrage nach Olefinen mit mehr als zwölf Kohlenstoffatomen deutlich geringer ist als die nach Olefinen acht oder zwölf Kohlenstoffatomen, wird die vorliegende Oligomerisierung von $C_4$-Olefinen dergestalt betrieben, dass im Wesentlichen $C_8$-Olefine und $C_{12}$-Olefine entstehen.

Ein industriell praktiziertes Verfahren zur Oligomerisierung von $C_4$-Olefinen ist der sogenannte "OCTOL-Prozess". Die ausführliche Beschreibung desselben findet sich in der Nicht-Patentliteratur, beispielsweise unter:

- B. Scholz: The HÜLS OCTOL Process: Heterogeneously catalyzed dimerization of n-butenes and other olefins. DGMK-Tagung in Karlsruhe, veröffentlicht in Erdöl, Ergas, Kohle, April 1989, Seiten 21 und 22.

- R.H. Friedlander, D.J. Ward, F. Obenaus, F. Nierlich, J. Neumeister: Make plasticizer olefins via n-butene dimerization. Hydrocarbon Processing, February 1986, Seiten 31 bis 33.

- F. Nierlich: Oligomerize for better gasoline. Hydrocarbon Processing, February 1992, Seiten 45 bis 46.

Innerhalb der Patentliteratur beschreibt beispielsweise DE102008007081A1 eine auf dem OCTOL-Prozess beruhende Oligomerisierung. EP1029839A1 beschreibt ein Verfahren zur Fraktionierung der in dem OCTOL-Prozess entstehenden $C_8$-Olefine.

Der OCTOL-Prozess wird in der Regel mehrstufig mit Hilfe einer Reaktorkaskade durchgeführt, die eine der Stufenanzahl entsprechenden Anzahl von hintereinandergeschalteten Reaktionszonen bzw. Reaktoren umfasst. Zwischen den einzelnen Reaktionszonen ist jeweils eine Destillationskolonne vorgesehen, welche die zuvor entstandenen Oligomere aus dem Oligomerisat von den nicht umgesetzten Butenen abtrennt und ausschleust. Die nicht umgesetzten Butene werden teilweise in die vorhergehende Oligomerisierung zurückgeführt und zum anderen Teil der nachfolgenden Oligomerisierung zugeführt. Der OCTOL-Prozess umfasst somit eine mehrfach durchlaufene Schrittsequenz, die sich zusammen setzt aus den vier Einzelschritten:

a) Bereitstellen eines Buten-haltigen Kohlenwasserstoffgemisches;
b) Oligomerisierung;
c) Abtrennen der nicht umgesetzten Butene aus dem Oligomerisat;
d) Rückführen eines Teils der nicht umgesetzten Butene in die vorhergehende Oligomerisierung bzw. des anderen Teils in die nachfolgende Oligomerisierung.

Durch die Rückführung der nicht umgesetzten Butene herrscht in den ersten Stufen stets ein Überangebot an Butenen, so dass in den einzelnen Oligomerisierungsstufen nie ein vollständiger Umsatz zu $C_8$-, $C_{12}$- und $C_{12+}$-Olefinen erreicht wird. Erst der gesamte, je nach Wirtschaftlichkeit zwei- bis sechsstufig durchgeführte OCTOL-Prozess kann insgesamt zu einem nahezu vollständigen Umsatz der eingesetzten Butene führen.

Ein weiteres Mehrstufen-Verfahren zur Oligomerisierung von $C_4$-Olefinen ist aus WO99/25668 bzw. aus DE10015002A1 bekannt. Hier wird eine Verdünnung der bereitgestellten Olefin-Ströme durch rückgeführte Butane praktiziert, um die Wärmeabfuhr aus der exothermen Reaktion über den Reaktoraustrag zu vereinfachen.

Je nachdem, auf welche Art und Weise sich die einzelnen n-Buten-Moleküle im Zuge der Oligomerisierung verbinden, erhält man ein Oligomerisat mit unterschiedlichem Verzweigungsgrad. Der Verzweigungsgrad wird durch den Isoindex beschrieben, welcher die mittlere Anzahl der Methylgruppen pro $C_8$-Molekül in dem Isomerengemisch angibt. Der Isoindex für Dibuten ist wie folgt definiert:

$$\text{Isoindex} = (\text{Gewichtsanteil Methylheptene} + 2* \text{ Gewichtsanteil Dimethylhexene}) / 100$$

So tragen n-Octene mit 0, Methylheptene mit 1 und Dimethylhexene mit 2 zum Isoindex eines Produktgemisches aus $C_8$-Olefinen bei. Je niedriger der Isoindex, umso weniger verzweigt sind die Moleküle innerhalb des Gemisches aufgebaut.

Für die Eigenschaften des Weichmachers spielt nun der Verzweigungsgrad des olefinischen Ausgangsgemisches, das für die Herstellung des Weichmacheralkohols verwendet wird, eine maßgebliche Rolle: Je höher die Linearität des $C_8$-Olefingemisches, desto besser sind die Eigenschaften des daraus hergestellten $C_9$-Weichmacheralkohols. Bei der Herstellung von Dibuten als Ausgangsprodukt für Weichmacheralkohole wird somit angestrebt, die Oligomerisierung so zu fahren, dass ein $C_8$-Produktgemisch erhalten wird, dessen Isoindex möglichst klein ist.

So wird beispielsweise in EP1029839A1 die Fraktionierung der Oligomerisate so eingerichtet, dass das abgetrennte $C_8$-Produktgemisch einen möglichst geringen Isoindex aufweist.

In WO99/25668A1 wird indes ein geringer Isoindex dadurch erreicht, dass man solche Mengen des vom Oligomerisat abgetrennten Butans und nicht umgesetzten Butens in die Oligomerisierung zurückführt, dass der maximale Gehalt an Oligomeren im umgesetzten Reaktionsgemisch 25 Gew.-% an keiner Stelle der Reaktorkaskade übersteigt.

Beide Verfahren nutzten als Ausgangsgemisch für die Oligomerisierung jeweils ein "Raffinat II", welches einen hohen Anteil an 1-Buten aufweist. Unter "Raffinat II" wird gemeinhin ein Butan/Buten-Gemisch verstanden, welches aus "C4-Schnitten" erhalten wird, die aus Steam-Crackern stammen und aus denen bereits Butadien und Isobuten abgetrennt wurden. So enthält typisches Raffinat II um die 30 Gew.-% 1-Buten.

Es lässt sich zeigen, dass ein hoher Anteil an 1-Buten im bereitgestellten Kohlenwasserstoffgemisch sich günstig auf die Linearität des Oligomerisats auswirkt.

Deshalb verwundert es nicht, dass die WO99/25668A1 ausgehend von dem Rohstoff Raffinat II $C_8$-Produktgemische herstellt, deren Isoindex kleiner ist als 1.

Auch Nierlich hebt im seinem oben zitierten Aufsatz "Oligomerize for better gasoline", hervor, dass sich Raffinat II besser als Ausgangsstoff für eine Oligomerisierung eigne als Raffinat III. "Raffinat III" erhält man durch Entfernung von 1-Buten aus Raffinat II, weswegen sein Gehalt an 1-Buten deutlich geringer ist als der von Raffinat II.

FCC-$C_4$ hält Nierlich erst nach vorheriger Hydroisomersierung für die Herstellung von Dibuten geeignet:

- F. Nierlich: Integrated tert. butyl Alcohol / Di-n-butenes Production from FCC C4's. Erdöl, Erdgas, Kohle. Jg. 103 (1987), Seiten 486 bis 489.

Aufgrund der inzwischen eingetretenen Rohstoffverknappung ist das petrochemisch hergestellte Raffinat II jedoch nicht mehr überall in großen Mengen und zu günstigen Konditionen verfügbar. So liefern alternative Rohstoffquellen $C_4$-Olefingemische, die teilweise fast gar kein 1-Buten mehr enthalten, sondern überwiegend 2-Buten.

Es war daher Aufgabe der Erfindung, ein Verfahren zur Oligomerisierung von Butenen anzugeben, mit welchem sich $C_8$-Olefine mit einem für die Weichmacherherstellung günstigen, niedrigen Isoindex selbst dann herstellen lassen, wenn der zu nutzende $C_4$-Strom das die Linearität begünstigende 1-Buten bloß in geringsten Mengen enthält. Darüber hinaus soll der gewünschte Prozess eine hohe Selektivität in Richtung der $C_8$-Olefine aufweisen, um eine möglichst große Menge der eingesetzten Butene zu Dimerisieren; die Reaktionen zu $C_{12}$- und $C_{12+}$-Oligomeren sollen nach Möglichkeit minimiert werden.

Es wurde nun gefunden, dass sich mit einem Oligomerisierungsverfahren der eingangs genannten Gattung das gesetzte Ziel erreichen lässt, wenn bei der erstmalig durchgeführten Schrittsequenz der unmittelbar über die erste Reaktionszone bilanzierte Umsatz von Butenen begrenzt wird auf einen ersten Grenzwert, der zwischen 5 und 40 Gew.-% liegt.

So konnte überraschend gezeigt werden, dass eine Begrenzung des Umsatzes in der ersten Stufe selbst dann zu $C_8$-Produktgemischen mit einem Isoindex kleiner als 1.2 führt, wenn in der erstmalig durchgeführten Schrittsequenz die auf die Konzentration an linearen Butenen bezogene Konzentration an 1-Buten in dem bereitgestellten Kohlenwasserstoffgemisch kleiner oder gleich der sich aus der Reaktionstemperatur der erstmalig durchgeführten Schrittsequenz ergebenen Gleichgewichtskonzentration an 1-Buten innerhalb der Fraktion der im erstmalig bereitgestellten Kohlenwasserstoffgemisch enthaltenen linearen Butene ist.

Dieser Befund ist deswegen überraschend, da bisher von keinem Buten-Oligomerisierungsverfahren berichtet wurde, welches selbst bei so geringen Konzentrationen an 1-Buten im Feed der Oligomerisierung noch zu einem $C_8$-Produktgemisch mit einem passablen Isoindex führt. So wurden bisher im Stand der Technik lediglich Oligomerisierungsverfahren angepriesen, die auf einen hohen Umsatz abzielten und deswegen möglichst hohe Reaktionstemperaturen anstrebten. Da in der Vergangenheit ausschließlich $C_4$-Ströme mit einem hohen Gehalt an 1-Buten genutzt wurden, konnten dennoch gering verzweigte $C_8$-Oligomerengemische erhalten werden.

Bevorzugt wird der unmittelbar über die erste Reaktionszone bilanzierte Umsatz in der ersten Stufe auf 10 bis 38 Gew.-% und ganz besonders bevorzugt auf einen ersten Grenzwert zwischen 10 und 36 Gew.-% begrenzt.

Unter Umsatz versteht sich der prozentuale Massenanteil der Butene, der innerhalb der betrachteten Bilanzgrenze zu Buten-Oligomeren umgesetzt wird.

Gegenstand der Erfindung ist mithin ein Verfahren zur Herstellung von $C_8$-Olefinen und von $C_{12}$-Olefinen durch Oligomerisierung von Butenen, welches die folgende Schrittsequenz umfasst:

a) Bereitstellen eines Kohlenwasserstoffgemisches enthaltend zumindest ein 2-Buten sowie mindestens ein weiteres, von diesem 2-Buten verschiedenes lineares Buten;

b) Oligomerisieren eines Teils der in dem Kohlenwasserstoffgemisch enthaltenden Butene zu $C_8$-Olefinen und zu $C_{12}$-Olefinen und gegebenenfalls zu $C_{12+}$-Olefinen durch Inkontaktbringen des Kohlenwasserstoffgemisches mit einem in einer Reaktionszone angeordneten Oligomerisierungskatalysator bei einer in der Reaktionszone herrschenden Reaktionstemperatur unter Erhalt eines die hergestellten Oligomere und die nicht umgesetzten Butene enthaltenden Oligomerisats;

c) Abtrennen der nicht umgesetzten Butene aus dem Oligomerisat;

d) optional Rückführen eines Teils der abgetrennten, nicht umgesetzten Butene in die vorhergehende Oligomerisierung;

bei welchem die Schrittsequenz mindestens einmal einschließlich des Schrittes "Rückführen" durchgeführt wird, wobei das Verfahren unter der Maßgabe durchgeführt wird, dass bei der erstmalig durchgeführten Schrittsequenz die auf die Konzentration an linearen Butenen bezogene Konzentration an 1-Buten in dem bereitgestellten Kohlenwasserstoffgemisch kleiner oder gleich der sich aus der Reaktionstemperatur der erstmalig durchgeführten Schrittsequenz ergebenen Gleichgewichtskonzentration an 1-Buten innerhalb der Fraktion der im erstmalig bereitgestellten Kohlenwasserstoffgemisch enthaltenden linearen Butene ist, und dass die hergestellten $C_8$-Olefine als ein $C_8$-Produktgemisch erhalten werden, dessen Isoindex kleiner ist als 1.2, wobei dieses Ziel dadurch erreicht wird, dass bei der erstmalig durchgeführten Schrittsequenz der unmittelbar über die erste Reaktionszone bilanzierte Umsatz von Butenen begrenzt wird auf einen ersten Grenzwert zwischen 5 und 40 Gew.-%, welcher bevorzugt zwischen 10 und 38 Gew.-% und ganz besonders bevorzugt zwischen 10 und 36 Gew.-% liegt.

Die Begrenzung des Umsatzes in den einzelnen Stufen erfolgt dabei erfindungsgemäß durch eine strenge Begrenzung der Reaktionstemperatur innerhalb der einzelnen Stufen und/oder eine strenge Kreislaufführung. Durch niedrige Reaktionstemperaturen und einen hohen Rückführanteil wird der unmittelbar an der Reaktionszone bilanzierte Umsatz von Butenen effektiv begrenzt.

Um trotz der starken Begrenzung des Umsatzes in der ersten Stufe einen über den Gesamtprozess ausreichend hohen Butenumsatz zu erzielen, ist eine entsprechend hohe Anzahl an Reaktionsstufen bzw. Schrittsequenzen erforderlich. Allerdings kann die Anzahl der Stufen nicht unendlich gewählt werden, da dies die Investitions- und Betriebskosten der Oligomerisierungsanlage signifikant erhöhen und damit den Gesamtprozess unwirtschaftlich werden ließe.

In manchen Investitionssituationen kann es daher wirtschaftlich sein, einen zweistufigen Prozess zu fahren, bei dem die Schrittsequenz "Bereitstellen, Oligomerisieren, Abtrennen und Rückführen" mindestens zweimal hintereinander durchgeführt, wobei bei der zweiten durchgeführten Schrittsequenz der unmittelbar an der zweiten Reaktionszone bilanzierte Umsatz von Butenen begrenzt wird auf einen zweiten Grenzwert zwischen 5 und 50 Gew.-%, welcher bevorzugt zwischen 10 und 40 Gew-% liegt.

Werden höhere Gesamtumsätze gefordert, kann das Verfahren dreistufig ausgeführt werden, so dass die Schrittsequenz "Bereitstellen, Oligomerisieren, Abtrennen und Rückführen" dreimal hintereinander durchgeführt wird, wobei bei der dritten durchgeführten Schrittsequenz der unmittelbar an der dritten Reaktionszone bilanzierte Umsatz von Butenen begrenzt wird auf einen dritten Grenzwert zwischen 5 und 65 Gew.-%, welcher bevorzugt zwischen 20 und 60 Gew-% liegt.

Eine noch bessere Einsatzstoffausnutzung wird bei einer vierstufigen Oligomerisierung erreicht, bei der die Schrittsequenz "Bereitstellen, Oligomerisieren, Abtrennen und Rückführen" viermal hintereinander durchgeführt wird, wobei in der vierten durchgeführten Schrittsequenz der unmittelbar an der vierten Reaktionszone bilanzierte Umsatz von Butenen begrenzt wird auf einen vierten Grenzwert zwischen 5 und 80 Gew.-% welcher bevorzugt zwischen 20 und 70 Gew-% liegt.

Eine fünfstufige Oligomerisierung, bei der die Schrittsequenz "Bereitstellen, Oligomerisieren, Abtrennen und Rückführen" fünfmal hintereinander durchgeführt wird, wird bei den derzeitigen Rohstoff- und Investitionskosten an der Grenze der Wirtschaftlichkeit der Anlage liegen, so dass heute eher eine vierstufige Verfahrensführung bevorzugt wird. Da in der Zukunft aber auch eine fünfstufige Oligomerisierung durchaus wirtschaftlich sein kann, sei darauf hingewiesen, dass in der fünften durchgeführten Schrittsequenz der unmittelbar an der fünften Reaktionszone bilanzierte Umsatz von Butenen auf einen fünften Grenzwert zwischen 5 und 95 Gew.-% begrenzt werden sollte, wobei der fünfte Grenzwert bevorzugt zwischen 20 und 80 Gew.% liegt.

Je nach Anzahl der gewählten Reaktionsstufen wird nach Durchführung aller Schrittsequenzen ein Gesamtumsatz an Butenen erzielt, der zwischen 5 und 100 Gew.-% liegt. Insbesondere wird die Stufenanzahl so gewählt, dass ein Gesamtumsatz zwischen 20 und 100 Gew.-% und ganz besonders bevorzugt zwischen 30 und 95 Gew.-% erzielt wird.

Wie bereits erwähnt, lässt sich der Umsatz effektiv durch eine Absenkung der Reaktionstemperatur in der jeweiligen Oligomerisierung begrenzen. Eine niedrige Reaktionstemperatur im Sinne der Erfindung liegt zwischen 40 °C und 140 °C. Bevorzugt wird die Reaktionstemperatur in den einzelnen Oligomerisierungen jedoch auf Werte zwischen 45 °C und 120 °C und ganz besonders bevorzugt zwischen 50 °C und 100 °C begrenzt.

Die Temperaturbegrenzung erfolgt vorzugweise durch Kühlung der Reaktionszone mittels eines externen Kühlmediums, welches selbst nicht in der Oligomerisierung gegenwärtig ist. Die Reaktionswärme wird über die Grenze der Reaktionszone (in der Regel die Reaktorwand) auf das Kühlmedium übertragen. Als Kühlmedium wird Wasser bevorzugt. Alternativ kann ein organisches Wärmeträgeröl wie beispielsweise Marlotherm® von Sasol Germany GmbH eingesetzt werden.

Bevorzugt wird das Verfahren so geführt, dass es hinsichtlich der hergestellten $C_8$-Olefine eine Selektivität von mehr als 80, insbesondere mehr als 82 und ganz besonders bevorzugt mehr als 84 Gew.-% erreicht. Dies bedeutet, dass mehr als die genannten Gewichtsanteile der hergestellten Oligomere $C_8$-Olefine sind. Die Reaktion in Richtung der $C_{12}$ und $C_{12+}$ -Olefine wird mithin minimiert, was letztendlich auch durch die niedrigen Temperaturen gelingt.

Ebenso wie die eingesetzten Butene in unterschiedlichen Strukturisomeren vorliegen, werden auch $C_8$-Olefine mit unterschiedlichen Strukturisomeren gebildet. Somit handelt es sich bei den hergestellten $C_8$-Olefinen in der Regel um n-Octene, Methylheptene und Dimethylhexene. Um einen für die Weichmacherherstellung günstigen Isoindex zu erzielen, sollten sich die hergestellten $C_8$-Olefine wie folgt zu 100 Gew.-% ergänzt zusammensetzen:

| | |
|---|---|
| n-Octene: | 10 bis 25 Gew.-%, bevorzugt 12 bis 20 Gew.-% und besonders bevorzugt 14 bis 20 Gew.-%; |
| Metylheptene: | 50 bis 80 Gew.-%, bevorzugt 55 bis 75 Gew.-% und besonders bevorzugt 60 bis 70 Gew.-%; |
| Dimethylhexene: | 10 bis 30 Gew.-%, bevorzugt 10 bis 25 Gew.-% und besonders bevorzugt 10 bis 20 Gew.-%. |

Mit einer derartigen Zusammensetzung wird dann ein $C_8$-Produktgemisch erzielt, dessen Isoindex unter 1.1 und ganz besonders bevorzugt unter 1.05 liegt.

Wie bereits erwähnt, zeichnet sich das erfindungsgemäße Verfahren dadurch aus, dass es die Verarbeitung von $C_4$-Strömen mit geringstem Gehalt an 1-Buten zu einem hoch linearen $C_8$-Produktgemisch gestattet. Bemerkenswert ist insbesondere, dass der Gehalt an 1-Buten gegenüber den 2-Butenen unterhalb des thermodynamischen Gleichgewichts der linearen Butene liegen kann; bestimmt nach den Bedingungen innerhalb der ersten Schrittsequenz.

Mithin können $C_4$-Einsatzgemische mit einem extrem geringen Anteil an 1-Buten genutzt werden. Die Erfindung erlaubt sogar die Verwendung von $C_4$-Einsatz-gemischen, die praktisch frei sind von 1-Buten. Damit ist das erfindungsgemäße Oligomerisierungsverfahren auf heutige Rohstoffsituationen besser angepasst als der herkömmliche OCTOL-Prozess.

Bevorzugt handelt es sich somit bei dem erstmalig bereitgestellten Kohlenwasserstoffgemisch um ein Eduktgemisch mit der folgenden, sich zu 100 Gew.-% ergänzenden Zusammensetzung:

| | |
|---|---|
| 1-Buten: | weniger als 10 Gew.-%, bevorzugt weniger als 5 Gew.-% und besonders bevorzugt weniger als 2 Gew.-%; |
| 2-Butene: | 20 bis 90 Gew.-% |
| Isobuten: | weniger als 5 Gew.-%, bevorzugt weniger als 1 Gew.-% |
| n-Butan: | weniger als 80 Gew.-% |
| Isobutan: | weniger als 80 Gew.-% |
| Sonstige: | weniger als 5 Gew.-%, bevorzugt weniger als 2 Gew.-% |

Ein solches Eduktgemisch könnte beispielsweise ein Raffinat III sein oder ein unveredelter $C_4$-Strom aus einem fluidkatalytischen Cracker.

Da das erfindungsgemäße Oligomerisierungsverfahren für den großindustriellen Einsatz bestimmt ist, ist es uneingeschränkt dafür tauglich, kontinuierlich betrieben zu werden. Dementsprechend wird es bevorzugt in einer Anlage durchgeführt, innerhalb welcher jeder durchgeführten Schrittsequenz genau eine Reaktionszone zugeordnet ist. Eine derartige Anlage stellt eine Reaktorkaskade dar. Die Reaktionszone kann dabei von einem oder von mehreren Reaktoren gebildet werden, die parallel oder seriell verschaltet sind. Entscheidend für die Definition einer Schrittsequenz im Sinne der vorliegenden Erfindung ist nicht die Anzahl der Reaktoren, sondern die Anzahl der Kombinationen aus Oligomerisierung und anschließender Abtrennung.

Es versteht sich von selbst, dass in jeder durchlaufenen Schrittsequenz mit Ausnahme der letzten Schrittsequenz der nicht rückgeführte Teil der abgetrennten, nicht umgesetzten Butene als Kohlenwasserstoffgemisch für die darauffolgende Schrittsequenz bereitgestellt wird.

Der nicht rückgeführte Teil der abgetrennten, nicht umgesetzten Butene der letzten Schrittsequenz wird dementsprechend nicht einer weiteren Oligomerisierung unterworfen, sondern anderweitig verwertet. Es bieten sich hier die folgenden Verwertungsmöglichkeiten an:

a) Vollhydrierung unter Erhalt eines Butangemisches;
b) Oxidative oder nichtoxidative Dehydrierung zu Butadien;
c) Hydroformylierung zu Pentanalen;
d) Oxidation insbesondere zu Maleinsäureanhydrid;
e) Metathese;
f) Hydratisierung zu Butanolen;
g) Alkylierung;
h) Isomerisierung;
i) Addition von Alkoholen zu Ethern;
j) Carbonylierung;
k) Cracken in einem Steam-Cracker oder einem fluidkatalytischen Cracker unter Erhalt von Kohlenwasserstoffen mit weniger als vier Kohlenstoffatomen;
l) Verbrennen unter Erhalt thermischer Energie.

Erfindungsgemäß findet hinter jedem Oligomerisierungsschritt eine Abtrennung der nicht umgesetzten Butene aus dem Oligomerisat statt, sodass die Oligomere zurückbleiben. Die Abtrennung erfolgt in an sich bekannter Weise destillativ. Dabei werden die nicht umgesetzten Butene über Kopf aus dem Oligomerisat abgetrennt, so dass die längerkettigen Oligomere im Sumpf verbleiben. Die Abtrennung lässt sich destillativ vergleichsweise einfach bewerkstelligen, da der Siedepunktunterschied zwischen den nicht umgesetzten $C_4$-Olefinen und den $C_8$-, $C_{12}$- und $C_{12+}$-Oligomeren recht groß ist.

Bevorzugt werden die in den einzelnen Schrittsequenzen erhaltenen Oligomere vereinigt und anschließend in einer weiteren Destillation aufgetrennt in das gewünschte $C_8$-Produktgemisch, in ein $C_{12}$-Produktgemisch und in ein $C_{12+}$-Produktgemisch. Die Vereinigung der einzelnen Produktgemische kann dabei bevorzugt auch in der hinter der letzten Oligomerisierung angeordneten Kolonne erfolgen.

Weitere Aspekte der vorliegenden Erfindung gehen aus der nun folgenden Beschreibung einiger Ausführungsformen hervor. Hierfür zeigen schematisch:

Figur 1:    einstufiges Verfahren;
Figur 2:    zweistufiges Verfahren;
Figur 3:    dreistufiges Verfahren;
Figur 4:    dreistufiges Verfahren mit Produktvereinigung vor der letzten Abtrennungskolonne.

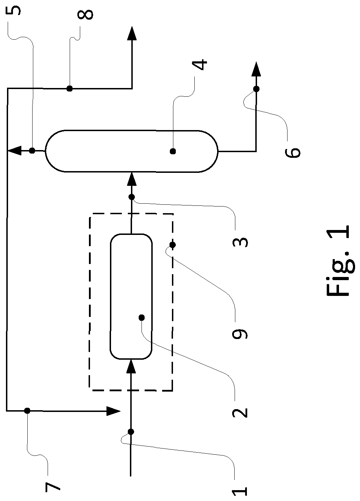

Figur 1 zeigt ein vereinfachtes Prozessfließbild eines erfindungsgemäßen Oligomerisierungsverfahrens. Ausgangsstoff ist ein Eduktgemisch 1, welches beispielsweise aus einem fluidkatalytischen Erdöl-Cracker stammt, einer Vorreinigung unterzogen worden sein kann und als kontinuierlicher Stoffstrom bereitgestellt wird. Bei dem Eduktgemisch 1 handelt es sich um ein Gemisch aus Kohlenwasserstoffen mit vier Kohlenstoffatomen enthaltend die $C_4$-Olefine 1-Buten, cis-2-Buten, trans-2-Buten und Isobuten sowie die $C_4$-Alkane n-Butan und Isobutan. Sonstige organische oder anorganische Begleitstoffe, die üblicherweise in $C_4$-Schnitten vorkommen, werden hier nicht betrachtet. Die Besonderheit des bereitgestellten Eduktgemisches 1 besteht darin, dass sein Gehalt an 1-Buten außergewöhnlich gering ist. Eduktgemisch 1 weist die folgende Zusammensetzung auf:

| | |
|---|---|
| 1-Buten: | weniger als 5 Gew.-%; |
| 2-Butene: | 20 bis 90 Gew.-% |
| Isobuten: | weniger als 1 Gew.-% |
| n-Butan: | weniger als 80 Gew.-% |
| Isobutan: | weniger als 80 Gew.-% |
| Sonstige: | weniger als 2 Gew.-% |

Eduktgemisch 1 wird in eine Reaktionszone 2 geleitet. Bei der dort herrschenden Reaktionstemperatur kommt es mit einem in der in der Reaktionszone 2 angeordneten Oligomerisierungskatalysator in Kontakt, sodass ein Teil der in dem Eduktgemisch 1 enthaltenen Butene miteinander zu Oligomeren reagieren und in einem Oligomerisat 3 aus der Reaktionszone 2 abgezogen werden. Das Oligomerisat ist ein Gemisch der gebildeten Oligomere, der nicht umgesetzten Butene und der in der Reaktion sich inert verhaltenen Bestandteile des Eduktgemisches wie den Butanen.

Zu den Oligomeren gehören $C_8$-Olefine wie n-Octene, Methylheptene und Dimethylhexene, welche durch die Oligomerisierung von zwei $C_4$-Olefinen entstehen. Oligomerisieren drei Butene oder ein Buten und ein zuvor gebildetes Octen miteinander, entstehen $C_{12}$-Olefine (Dodecene). Vier miteinander oligomerisierende Butene oder zwei Butene und ein zuvor gebildetes Octen oder zwei zuvor gebildete Octene oder ein Buten und ein zuvor gebildetes Dodecen führen zu $C_{16}$-Olefinen.

Bei der in der Reaktionszone 2 durchgeführten Oligomerisierung werden überwiegend $C_8$-Olefinen gebildet; $C_{12}$-Olefine sind das größte Nebenprodukt. Die Olefine mit mehr als zwölf Kohlenstoffatomen werden nur zu vergleichsweise geringen Anteilen gebildet und als $C_{12+}$-Olefine zusammen gefasst.

Typischerweise setzt sich das Oligomerisat wie folgt zu 100 Gew.-% ergänzt zusammen:

| | |
|---|---|
| Butane | weniger als 80 Gew.-% |
| 1-Buten | 1 bis 5 Gew.-% |
| 2-Butene | 10 bis 80 Gew.-% |
| n-Octene | 1 bis 10 Gew.-% |
| Methyl heptene | 5 bis 40 Gew.-% |
| Dimethylhexene | 1 bis 15 Gew.-% |
| $C_{12}$-Olefine | 1 bis 10 Gew.-% |
| $C_{12+}$-Olefine | 0.1 bis 2 Gew.-% |

Bei dem in den Figuren nicht dargestellten Oligomerisierungskatalysator handelt es sich um einen heterogenen, Nickel-haltigen Katalysator. Bevorzugt wird ein Trägerkatalysator eingesetzt umfassend ein Trägermaterial wie beispielsweise Siliciumdioxid oder Aluminiumoxid oder Mischungen daraus oder Alumosilikate oder Zeolithe. Die Träger können Schwefel in Form von Sulfat, Sulfid oder anderen Verbindungsarten enthalten. Geeignete Oligomerisierungskatalysatoren sind in der Fachliteratur bekannt und werden beispielsweise in DE 4339713 A1 oder in WO 2001/37989 A2 oder in WO 2011/000697 A1 beschrieben.

Für die Herstellung der eingesetzten Nickel-Trägerkatalysatoren gibt es verschiedene Wege. Beispielsweise können solche Katalysatoren durch gemeinsames Fällen von Nickelverbindungen und Trägermaterial (nämlich Aluminium- und/oder Siliziumverbindungen), filtrieren und anschließendes Tempern hergestellt werden. Eine weitere Möglichkeit besteht darin, Nickelverbindungen auf ein geeignetes Trägermaterial aufzubringen, beispielsweise durch Imprägnieren oder Aufsprühen und anschließende Kalzinierung. Zur Herstellung der Katalysatoren nach dem Imprägnierverfahren können Nickelverbindungen wie beispielsweise Nickelnitrat, Nickelchlorid oder Aminkomplexe eingesetzt werden. Als Trägermaterialien werden bevorzugt kommerziell erhältliche Katalysatorträger wie beispielsweise amorphe Silizium-Aluminium-Mischoxide mit der Typenbezeichnung "Grace DAVICAT" erhältlich bei der Fa. Grace oder Zeolithe (z.B. MCM41) von Mobile Oil eingesetzt.

Ganz besonders bevorzugt werden Titan-freie Träger bzw. Trägerkatalysatoren eingesetzt, die im Wesentlichen aus Nickeloxid, Aluminiumoxid und Siliziumoxid bestehen. Vorzugsweise enthalten diese Katalysatoren 5 bis 50 Massen-% Nickel, insbesondere 10 bis 30 Massen-% Nickel. Die Gehalte an Aluminium liegen im Bereich von 5 bis 30 Massen-%, insbesondere im Bereich von 7 bis 20 Massen-%. Die Anteile an Silizium liegen im Bereich von 10 bis 40 Massen-%, wobei der Bereich von 20 bis 30 Massen-% besonders bevorzugt ist. Die angegebenen Massenanteile beziehen sich auf den gesamten Metallanteil. Als weitere Komponenten können diese Katalysatoren 0.1 bis 2 Massen-% Alkalioxid, Erdalkalioxid, Lanthanoxid oder Oxide der seltenen Erden und ggf. Formgebungshilfsmittel enthalten.

Makroskopisch wird der erfindungsgemäß verwendete Nickelkatalysator in einer Gestalt eingesetzt, in der er einen geringen Strömungswiderstand bietet. Bevorzugt liegt der Oligomerisierungskatalysator in Form von Formkörpern wie Granalien, Pellets, Tabletten, Zylinder, Kugeln, Strangsextrudate oder Ringen vor.

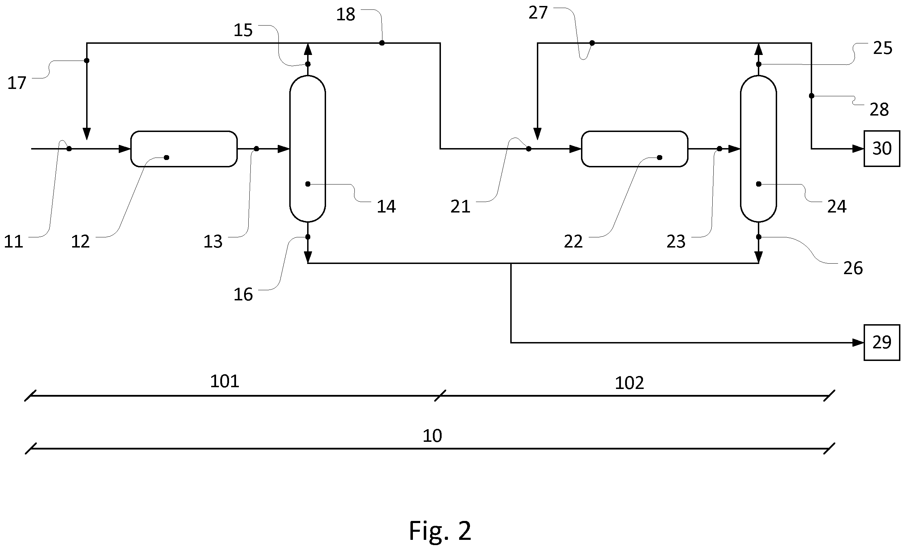

Apparatetechnisch wird die Reaktionszone 2 vorzugsweise als Rohrbündelreaktor bzw. als serielle oder parallele Verschaltung mehrerer Reaktoren durchgeführt. Selbst dann, wenn die Oligomerisierung in mehreren hintereinander verschalteten Reaktoren durchgeführt wird, handelt es sich dabei um einen einzigen Oligomerisierungsschritt, da in der Terminologie der vorliegenden Erfindung ein Oligomerisierungsschritt stets durch einen Abtrennschritt abgeschlossen wird. Dazu später mehr anhand der Figur 2.

Der vorzugsweise eingesetzte Rohrbündelreaktor umfasst eine Vielzahl von durchströmten Rohren, die mit Katalysator befüllt sind. Am Anfang der Rohre strömt das Eduktgemisch 1 gegebenenfalls vermischt mit Rückführstrom 7 ein, am Ende der Rohre wird das Oligomerisat 3 abgezogen. Die im Zuge der exothermen Oligomerisierungsreaktion entstehende Reaktionswärme wird bevorzugt nicht über das abströmende Oligomerisat 3, sondern über ein externes Kühlmedium (nicht dargestellt) abgeführt. Das Kühlmedium strömt dabei durch einen die Rohrbündel umgebenden Mantel, sodass ein Wärmeaustausch zwischen dem Reaktionsgemisch und dem Kühlmedium ohne Materieaustausch stattfinden kann. Das Kühlmedium nimmt an der Reaktion nicht teil; der Rohrbündelreaktor erfüllt demnach auch die Funktion eines Wärmetauschers. Als Kühlmedium eignet sich einfachstenfalls Wasser oder ein organisches Wärmeträgerfluid wie beispielsweise Marlotherm® von Sasol Germany GmbH.

Die Einstellung der Reaktionstemperatur innerhalb der Oligomerisierung mittels des Kühlmediums ist deshalb von besonderem Interesse, da gemäß der Erfindung die Begrenzung der Reaktionstemperatur eine wichtige Maßnahme zur Begrenzung des Umsatzes darstellt. So ist die Reaktionstemperatur ganz besonders bevorzugt auf eine vergleichsweise niedrigen Wert zwischen 50 und 100 °C zu begrenzen, was durch den Einsatz des externen Kühlmediums ermöglicht wird.

Der Druck innerhalb der Reaktionszone wird so gewählt, dass die gegenwärtigen $C_4$-Kohlenwasserstoffe in flüssiger Phase vorliegen. Der Druck wird dementsprechend zwischen 0.1 bis 70 MPa eingestellt, bevorzugt von 0.1 bis 10 und ganz besonders bevorzugt von 0.5 bis 4 MPa.

Die spezifische Katalysatorbelastung (WHSV) beträgt zwischen 0.1 und 5 min$^{-1}$ vorzugsweise 0.2 und 3 min$^{-1}$.

Das aus der Reaktionszone 2 abgezogene Oligomerisat 3 wird nun in einer Trenneinrichtung in Gestalt einer Destillationskolonne 4 eingebracht und darin in an sich bekannter Weise destillativ aufgetrennt in einen Kopfstrom 5 enthaltend die in der Oligomerisierung nicht umgesetzten Butene und die inerten Butane, sowie in einen Sumpfstrom 6 enthaltend die hergestellten Oligomere. Die Destillation erfolgt vorzugsweise bei einem Druck von 0.1 bis 1 MPa, bevorzugt bei 0.2 bis 0.5 MPa. Aufgrund des erheblichen Unterschieds im Molekulargewicht und dem daraus resultierenden deutlichen Unterschied in den Siedepunkten zwischen den über Kopf abgezogenen $C_4$-Kohlenwasserstoffen und den Oligomeren mit acht und mehr Kohlenwasserstoffen am Sumpf gelingt die Trennung innerhalb der Destillationskolonne 4 mit vergleichsweise geringem technischen Aufwand, so dass sich nähere Ausführungen dazu erübrigen. Weitere Informationen zur Gestaltung der destillativen Aufreinigung von Oligomerisaten finden sich in EP1029839A1.

Der die nicht umgesetzten Butene enthaltende Kopfstrom 5 wird aufgeteilt in einen Rückführstrom 7 und in einen Transferstrom 8. Der Rückführstrom 7 wird mit dem ursprünglich bereitgestellten Eduktstrom 1 vermischt und erneut der Oligomerisierung 2 zugeführt. Der Transferstrom 8 wird einer weiteren Verwertung der darin enthaltenen Butene und Butane zugeführt (nicht dargestellt). Der Mengenanteil des rückgeführten Kopfproduktes, also das Teilungsverhältnis der Ströme 7 zu 5 bzw. 8 zu 5, stellt neben der Reaktionstemperatur einen weiteren Parameter zur Begrenzung des Umsatzes innerhalb der Reaktionszone 2 dar. Erfindungsgemäß wird nämlich der Umsatz innerhalb der Reaktionszone 2 begrenzt auf einen ersten Grenzwert zwischen 5 und 40 Gew.-%. Der genannte Umsatz wird dabei unmittelbar an der Reaktionszone 2 bilanziert, also innerhalb der gestrichelt eingezeichneten Bilanzgrenze 9. Gemeint ist somit der Umsatz "per pass" bezogen auf den Reaktorzulauf, der sich additiv aus dem Frischzulauf 1 und dem (optionalen) Rückführstrom 7 zusammensetzt.

Der Umsatz innerhalb der Bilanzgrenze 9 wird einerseits begrenzt durch die Limitierung der Reaktionstemperatur durch eine entsprechende Kühlung der Reaktionszone 2 über das Kühlmedium sowie über die Größe des Rückführstroms 7.

Am Sumpf der Destillationskolonne 4 wird der Sumpfstrom 6 abgezogen, welcher die hergestellten Oligomere enthält. Diese werden noch entsprechend hinsichtlich ihres Molekulargewichtes getrennt (in Figur 1 nicht dargestellt).

Das in Figur 1 dargestellte Oligomerisierungsverfahren stellt die einfachste Ausführungsform der Erfindung dar, bei der die Schrittsequenz Bereitstellen, Oligomerisieren, Abtrennen und optional Rückführen nur ein einziges Mal durchlaufen wird. Aufgrund der erfindungsgemäßen Begrenzung des Umsatzes innerhalb der gestrichelt eingezeichneten Bilanzgrenze 9 auf maximal 40 % kann ohne Rückführstrom 7 nur ein über den gesamten Prozess bilanzierter Gesamtumsatz von 40 % erreicht werden. Um den Gesamtumsatz zu steigern wird die besagte Schrittsequenz vorzugsweise mit Rückführstrom 7 und/oder mehrfach hintereinander ausgeführt, zum Beispiel zweimal wie in Figur 2 dargestellt.

In dem zweistufigen Verfahren der Figur 2 wird die in Figur 1 dargestellte Schrittsequenz zweimal hintereinander durchlaufen, so dass sich der Gesamtprozess 10 in eine erste Schrittsequenz 101 und eine zweite Schrittsequenz 102 unterteilt. Da es sich um einen kontinuierlich betriebenen Gesamtprozess 10 handelt, sind die in jeder Schrittsequenz benötigten Apparate entsprechend doppelt vorhanden und seriell verschaltet. Man spricht dabei von einer Reaktorkaskade.

Innerhalb der erstmalig durchlaufenen Schrittsequenz 101 wird erstmalig ein $C_4$-Kohlenwasserstoffgemisch als Eduktgemisch 11 bereitgestellt sodann in einer ersten Reaktionszone 12 oligomerisiert und das dabei erhaltene erste Oligomerisat 13 in einer ersten Destillationskolonne 14 aufgetrennt in einen ersten Kopfstrom 15 und einen ersten Sumpfstrom 16. Ein Teil des ersten Kopfstromes 15 wird die vorhergehende Oligomerisierung 12 zurückgeführt, um dort bisher nicht umgesetzte Butene nun umzusetzen, währenddessen der andere Teil als erster Transferstrom 18 in die zweite Schrittsequenz 102 überführt wird. Er dient darin als bereitgestelltes Kohlenwasserstoffgemisch 21 für die zweite Stufe der Oligomerisierung, die in einer zweiten Reaktionszone 22 erfolgt. Das darin erhaltene zweite Oligomerisat 23 wird in einer zweiten Destillationskolonne 24 wiederum aufgetrennt in einen zweiten Kopfstrom 25 und einen zweiten Sumpfstrom 26. Der Kopfstrom 25 der zweiten Destillationskolonne 24 wird aufgeteilt in einen zweiten Rückführstrom 27 und einen zweiten Transferstrom 28.

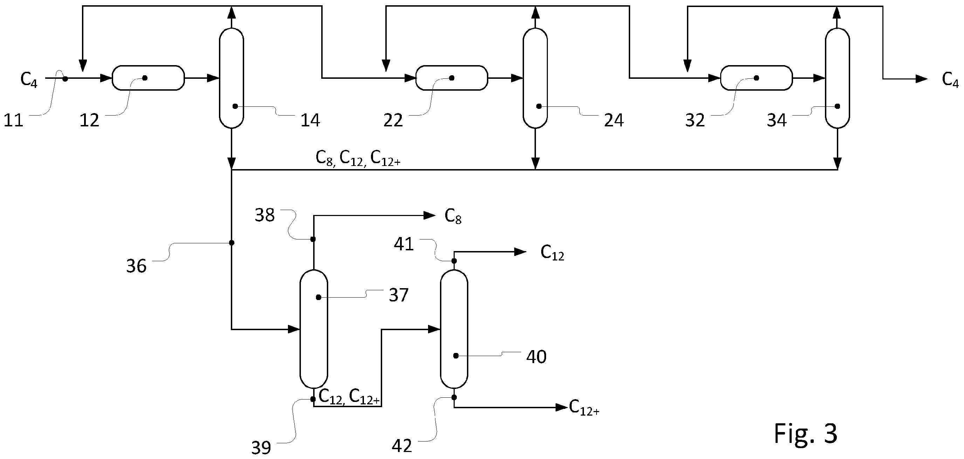

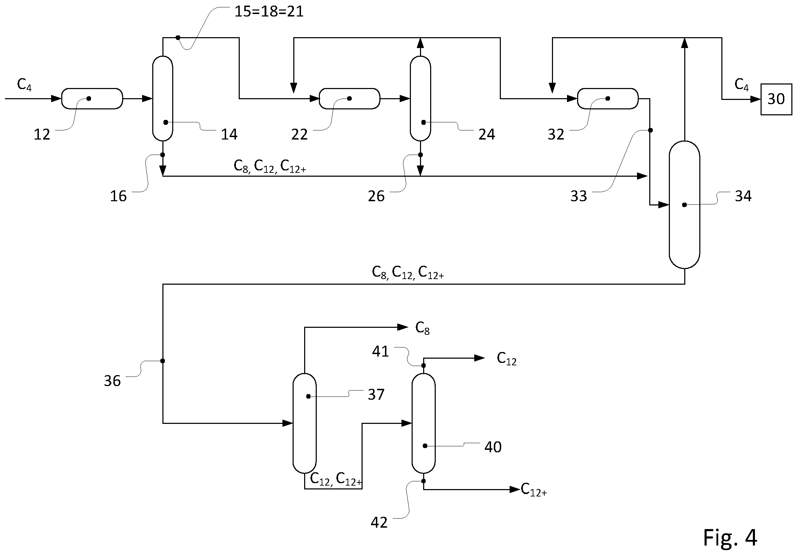

Der Sumpfstrom 26 der zweiten Destillationskolonne 24 wird mit dem ersten Sumpfstrom 16 vereinigt und einer gemeinsamen Fraktionierung 29 der darin enthaltenen Oligomeren zugeführt. Mögliche Ausführungsformen der Fraktionierung 29 werden anhand der Figuren 3 und 4 erläutert.

Der in der zweiten und damit letzten Schrittsequenz 102 nicht rückgeführte Teil der abgetrennten, nicht umgesetzten Butene, werden nicht mehr oligomerisiert und mit dem zweiten Transferstrom 28 einer Verwertung 30 zugeführt. Die Verwertung 30 besteht einfachstenfalls in dem Verbrennen des nicht rückgeführten Kopfproduktes der letzten Kolonne 24. Sofern in Anlagennähe ein fluidkatalytischer Cracker oder Steam-Cracker verfügbar ist, bietet es sich an, den Strom 28 zurück in den Cracker zu führen und ihn dort in Kohlenwasserstoffe mit weniger als vier Kohlenstoffatomen aufzutrennen. Ist ein solcher Cracker nicht verfügbar, können die in dem nicht rückgeführten zweiten Transferstrom 28 enthaltenen Butene zu Butanen vollhydriert werden, sodass in der Verwertung 30 ein Butangemisch anfällt, dass sich als Treibgas oder Brenngas für den Privatgebrauch eignet. Darüber hinaus bestehen weitere stoffliche Verwertungsmöglichkeiten für die nicht umgesetzten Butene, deren Wirtschaftlichkeit von der Abnahmesituation und der Zusammensetzung des die Oligomerisierung verlassenden Stoffstroms 28 abhängig ist.

In Figur 3 ist eine weitere Ausführungsform der Erfindung dargestellt, in welcher das erstmalig bereitgestellte $C_4$-Kohlenwasserstoffgemisch 11 durch dreimaliges Durchlaufen der Schrittsequenz in insgesamt drei Reaktionszonen 12, 22, 32 oligomerisiert wird. Jeder Reaktionszone 12, 22, 32 ist dabei eine eigene Destillationskolonne 14, 24, 34 zugeordnet, in welcher die in der jeweils zuvor durchlaufenen Oligomerisierung hergestellten Oligomere abgetrennt werden. Hierzu werden die Sumpfströme der Destillationskolonnen 14, 24, 34 vereinigt und einer gemeinsamen Fraktionierung zugeführt.

Zur Fraktionierung der hergestellten Oligomere werden die vereinigten Sumpfströme 36 zunächst in eine $C_8$-Kolonne 37 geleitet. Hierin erfolgt die destillative Abtrennung des eigentlichen Zielproduktes des Prozesses, einem $C_8$-Produktgemisch über Kopf. Das $C_8$-Produktgemisch besteht fast ausschließlich aus $C_8$-Olefinen mit der folgenden, sich zu 100 Gew.-% ergänzenden Zusammensetzung:

| n-Octene: | 10 bis 25 Gew.-% |
|---|---|
| Methylheptene: | 50 bis 80 Gew.-% |
| Dimethylhexene: | 10 bis 30 Gew.-% |

Der Isoindex des dort erhaltenen $C_8$-Produktgemisches 38 ist kleiner als 1.1.

Etwa 80 % aller gebildeten Buten-Oligomere sind Di-Butene und finden sich in dem $C_8$-Produktgemisch, sodass die Selektivität des Prozesses hinsichtlich des gewünschten Zielprodukts ($C_8$-Olefine) recht groß ist.

Das Sumpfprodukt 39 der $C_8$-Kolonne 37 enthaltend die hergestellten Oligomeren mit zwölf und mehr als zwölf Kohlenstoffatomen wird einer $C_{12}$-Kolonne 40 zugeführt und dort aufgetrennt in ein $C_{12}$-Produktgemisch 41, welches über Kopf abgezogen wird und ein $C_{12+}$-Produktgemisch 42 am Sumpf der $C_{12}$-Kolonne 40.

Auf die im $C_{12}$-Produktgemisch 41 enthaltenen $C_{12}$-Olefine entfallen etwa 7 bis 17 % der gebildeten Oligomere. Das durchaus im nennenswerten Umfang gebildete $C_{12}$-Produktgemisch kann zur Herstellung von Waschmittelalkoholen verwendet werden.

Die in dem $C_{12+}$-Produktgemisch enthaltenen Olefine mit mehr als zwölf Kohlenstoffatome können hydriert und leichtem Heizöl bzw. dem Dieselkraftstoff zugemischt werden.

Figur 4 zeigt eine weitere erfindungsgemäße Variante eines dreistufigen Verfahrens. Bei der in Figur 4 dargestellten Ausführungsform entfällt innerhalb der erstmalig durchgeführten Schrittsequenz der optionale Schritt "Rückführen". Mithin wird der gesamte Kopfstrom 15 der ersten Destillationskolonne 14 als erster Transferstrom 18 in die zweite Schrittsequenz überführt und damit das für die zweite Stufe benötigte Kohlenwasserstoffgemisch 21 bereitgestellt.

Im Sinne der Erfindung könnte die Rückführung auch in einer anderen Stufe als in der ersten entfallen oder es können sogar mehrere Schrittsequenzen ohne den Schritt "Rückführen" durchgeführt werden. Jedoch ist in mindestens einer Schrittsequenz eine Rückführung vorzusehen. Bei einem einstufigen Verfahren wird die Rückführung denknotwendig in der ersten und einzigen Schrittsequenz durchgeführt.

Gegenüber der in Figur 3 dargestellten Ausführungsform eines dreistufigen Verfahrens ist die Fraktionierung der hergestellten Oligomere hier anders ausgeführt: So werden die Sumpfströme 16 und 26 der ersten und zweiten Destillationskolonne 14, 24 mit dem Oligomerisat 33 der dritten Stufe vereinigt und dann der dritten Destillationskolonne 34 zugeführt. Die dritte Destillationskolonne 34 ist entsprechend größer dimensioniert als in der in Figur 3 dargestellten Ausführungsform. Der Sumpfstrom 36 der dritten Destillationskolonne 34 entspricht dann den vereinigten Sumpfströmen 36 der in Figur 3 gezeigten Ausführungsform. Die Fraktionierung der Oligomere aus den vereinigten Sumpfströmen 36 entspricht der in Figur 3 dargestellten Ausführungsform.

Beispiel 1 (nicht erfindungsgemäß)

Das nicht erfindungsgemäße Beispiel 1 wurde in Anlehnung an WO 99/25668 A1 in einem weitgehend adiabatisch betriebenen Rohreaktor mit folgenden Maßen durchgeführt: Länge 2.0 m, Innendurchmesser 32.8 mm. Die Reaktion wurde bei einem Absolutdruck von 3 MPa in der Flüssigphase durchgeführt.

Als Einsatzstoff wurde ein Kohlenwasserstoffgemisch verwendet, enthaltend folgende, sich zu 100 Gew.-% ergänzende Komponenten:

| 1-Buten | 25 % |
|---|---|
| 2-Buten | 51 % |
| Isobuten | weniger 1 % |
| Isobutan | weniger 2 % |
| n-Butan | mehr als 21 % |

Das Gemisch enthielt somit entgegen der Lehre der vorliegenden Erfindung eine 1-Buten-Menge oberhalb der Konzentration von 1-Buten, die sich im thermodynamischen Gleichgewicht der n-Butene bei Reaktionstemperatur (hier Reaktoreingangstemperatur 60 °C) einstellt. Bei einer Temperatur von 60°C liegt dieser Wert bei etwa 4.1 % im Gesamtgemisch, bzw. bei 5.4 % innerhalb der n-Buten-Fraktion.

Ein Teil des Stromes der nicht umgesetzten Butene wurde in den Reaktor zurückgeführt (Recycle), die rückgeführten Mengen wurden entsprechend der Lehre der WO 99/25668 A1 so gewählt, dass die Oligomerenkonzentration an keinem Punkt des Reaktors 25 % überscheitet und im Reaktoraustrag 10 % nicht unterschritten werden. Die einzelnen Konzentrationen der Oligomere sind Tabelle 1 zu entnehmen.

Als Katalysator wurde ein Material verwendet, welches gemäß Beispiel 1 der WO 2011/000697 A1 hergestellt und nach Beispiel 4 derselben Veröffentlichung nachbehandelt worden war.

Der Produktstrom wurde mittels Gaschromatographie (GC) auf seine Zusammensetzung untersucht. Dazu wurde zur Identifizierung der Octen-Skelettisomeren eine hydrierende GC-Analytik verwendet, bei der die oligomeren Olefine zunächst zu Alkanen hydriert werden. Die so erhaltenen Alkane werden dann chromatographisch getrennt und detektiert. Man kann zwischen drei relevanten $C_8$-Isomeren differenzieren: n-Octan (gebildet aus n-Octenen), Methylheptan (gebildet aus Methylheptenen) und Dimethylhexan (gebildet aus Dimethylhexenen). Die Zusammensetzung des hydrierten $C_8$-Gemisches ist in Tabelle 1 zusammengefasst.

**Beispiele 2 bis 5 (nicht erfindungsgemäß)**

Die Beispiele wurden in Anlehnung an WO 99/25668 A1 in einem weitgehend adiabatisch betriebenen Rohrreaktor mit folgenden Maßen durchgeführt: Länge 2.0 m, Innendurchmesser 32.8 mm. Die Reaktion wurde bei einem Absolutdruck von 3 MPa in der Flüssigphase durchgeführt. Als Einsatzstoffe wurden zwei Kohlenwasserstoffgemische mit unterschiedlichem 1-Buten/2-Buten-Verhältnis aber konstanter n-Buten-Gesamtmenge eingesetzt. Die Konzentrationen der n-Butene sind Tabelle 1 zu entnehmen. Die Gemische enthielten darüber hinaus folgende sich zu 100 Gew.-% ergänzende Komponenten:

| Isobutan | weniger 2 % |
| n-Butan | mehr als 21 % |
| Isobuten | weniger 1 % |

Alle Gemische enthielten somit 1-Buten-Mengen unterhalb der 1-Buten-Konzentration, die sich im thermodynamischen Gleichgewicht der n-Butene bei der hier auf 60°C eingestellten, am Reaktoreingang gemessenen Reaktionstemperatur einstellt.

Ein Teil des Stromes der nicht umgesetzten Butene wurde, wie in Beispiel 1 beschrieben, in den Reaktor zurückgeführt (Recycle).

Als Katalysator wurde das gleiche Material wie in Beispiel 1 verwendet.

Tabelle 1: Analytik der Beispiele 1 bis 5

| Beispiel-Nr. | 1 | 2 | 3 | 4 | 5 |
| --- | --- | --- | --- | --- | --- |
| Frischzulauf [g/h] | 850 | 850 | 850 | 850 | 850 |
| Konzentration 1-Buten im Frischzulauf [wt%] | 25.0 | 0.5 | 0.5 | 4.0 | 4.0 |
| Konzentration 2-Buten im Frischzulauf [wt%] | 51.0 | 75.5 | 75.5 | 72.0 | 72.0 |
| Recyclemenge [g/h] | 1500 | 4200 | 1500 | 4200 | 1500 |
| Eingangstemperatur [°C] | 60.0 | 60.0 | 60.0 | 60.0 | 60.0 |
| Ausgangstemperatur [°C] | 120.4 | 96.3 | 119.3 | 96.6 | 119.0 |
| per-Pass-Umsatz [wt%] | 50.7 | 22.8 | 45.9 | 23.4 | 46.4 |
| Konzentration Oligomere im Reaktoraustrag [wt%] | 23.7 | 10.3 | 22.9 | 10.4 | 23.0 |
| Gesamtumsatz [%] | 86.5 | 80.3 | 83.3 | 81.1 | 83.7 |
| $C_8$-Selektivität [%] | 80.6 | 86.9 | 81.9 | 86.7 | 81.7 |
| Massenanteile am hydrierten $C_8$-Gemisch [wt%] | | | | | |
| n-Octan | 17.2 | 11.7 | 12.6 | 12.2 | 13.1 |
| Methylheptan | 62.8 | 68.0 | 61.6 | 68.0 | 62.4 |
| Dimethylhexan | 18.9 | 19.2 | 24.7 | 18.8 | 23.4 |
| Isoindex | 1.017 | 1.075 | 1.123 | 1.067 | 1.105 |

**Beispiele 6 bis 12 (erfindungsgemäß)**

Die Beispiele 6 bis 12 wurden in einer dreistufigen Reaktorkaskade aus weitgehend isotherm betriebenen Rohreaktoren mit folgenden Maßen durchgeführt: Länge 2.0 m, Innendurchmesser 32.8 mm. Die Oligomerisierung wurde jeweils bei einem Absolutdruck von 3 MPa in der Flüssigphase durchgeführt. Als Einsatzstoffe wurden zwei Kohlenwasserstoffgemische mit unterschiedlichem 1-Buten/2-Buten-Verhältnis aber konstanter n-Buten-Gesamtmenge eingesetzt. Die Konzentrationen der n-Butene sind Tabelle 2 zu entnehmen. Die Gemische enthielten darüber hinaus folgende sich zu 100 Gew.-% ergänzende Komponenten:

| Isobutan | weniger 2 % |
| n-Butan | mehr als 21 % |
| Isobuten | weniger 1 % |

Alle Gemische enthielten somit 1-Buten-Mengen unterhalb der 1-Buten-Konzentration, die sich im thermodynamischen Gleichgewicht der n-Butene bei Reaktionstemperatur (hier Reaktoreingangstemperatur 60 °C) einstellt, bzw. waren im Rahmen der analytischen Nachweisbarkeit frei von 1-Buten (Beispiel 12).

Es wurde der gleiche heterogene Nickel-Katalysator wie in den Bespielen 1 bis 5 verwendet.

Hinter jeder Reaktionsstufe wurden die Oligomere von den Butanen und nicht umgesetzten Butenen abgetrennt und wie in Beispiel 1 beschrieben auf ihre Zusammensetzung analysiert. Ein Teil des Butane und nicht umgesetzte Butene enthaltenden Stromes wurde in den vorhergehenden Reaktor zurückgeführt (Mengen siehe Tabelle 2). Der nicht rückgeführte Teil dieses Gemisches wurde als Frischzulauf für die folgende Reaktionsstufe verwendet (sofern vorhanden).

| Beispiel-Nr. | 6 | 7 | 8 | 9 | 10 | 11 | 12 |
|---|---|---|---|---|---|---|---|
| Frischzulauf [g/h] | 850 | 850 | 850 | 850 | 850 | 850 | 850 |
| Konzentration 1-Buten im Frischzulauf [wt%] | 0.5 | 0.5 | 0.5 | 4.0 | 4.0 | 4.0 | 0.0 |
| Konzentration 2-Buten im Frischzulauf [wt%] | 75.5 | 75.5 | 75.5 | 72.0 | 72.0 | 72.0 | 76.0 |
| Recyclemenge [g/h] | | | | | | | |
|   1. Stufe | 100 | 100 | 100 | 100 | 100 | 100 | 100 |
|   2. Stufe | - | 100 | 100 | - | 100 | 100 | 100 |
|   3. Stufe | - | - | 100 | - | - | 100 | 100 |
| Eingangstemperatur [°C] | | | | | | | |
|   1. Stufe | 60.0 | 60.0 | 60.0 | 60.0 | 60.0 | 60.0 | 60.0 |
|   2. Stufe | - | 60.0 | 60.0 | - | 60.0 | 60.0 | 60.0 |
|   3. Stufe | - | - | 70.0 | - | - | 70.0 | 70.0 |
| Ausgangstemperatur [°C] | | | | | | | |
|   1. Stufe | 60.9 | 60.9 | 60.9 | 60.9 | 60.9 | 60.9 | 60.9 |
|   2. Stufe | - | 60.6 | 60.6 | - | 60.6 | 60.6 | 60.6 |
|   3. Stufe | - | - | 70.4 | - | - | 70.4 | 70.4 |
| per-Pass-Umsatz [wt%] | | | | | | | |
|   1. Stufe | 35.1 | 35.1 | 35.1 | 34.7 | 34.7 | 34.7 | 35.1 |
|   2. Stufe | - | 42.5 | 42.5 | - | 42.5 | 42.5 | 42.5 |
|   3. Stufe | - | - | 59.2 | - | - | 59.2 | 59.2 |
| Konzentration Oligomere im Reaktoraustrag [wt%] | | | | | | | |
|   1. Stufe | 26.4 | 26.4 | 26.4 | 26.1 | 26.1 | 26.1 | 26.4 |
|   2. Stufe | - | 27.2 | 27.2 | - | 27.2 | 27.2 | 27.2 |
|   3. Stufe | - | - | 26.9 | - | - | 27.0 | 26.9 |
| Gesamtumsatz [%] | 38.7 | 68.2 | 89.4 | 38.4 | 67.9 | 89.3 | 89.4 |
| $C_8$-Selektivität [%] | 84.7 | 83.9 | 82.6 | 84.7 | 83.9 | 82.7 | 82.6 |

Tabelle 2: Analytik der Beispiele 6 bis 11

| Massenanteile am hydrierten C8-Gemisch [wt%] | | | | | | | |
|---|---|---|---|---|---|---|---|
|   n-Octan | 14.4 | 14.7 | 14.5 | 15.2 | 15.1 | 14.8 | 14.5 |
|   Methylheptan | 66.6 | 66.8 | 66.3 | 67.8 | 67.5 | 66.9 | 66.2 |
|   Dimethylhexan | 18.9 | 18.5 | 19.1 | 17.0 | 17.4 | 18.3 | 19.3 |
| Isoindex | 1.045 | 1.039 | 1.047 | 1.019 | 1.024 | 1.035 | 1.049 |

**Schlussfolgerung**

Der Vergleich der nicht erfindungsgemäßen Beispiele 1 und 2 belegt, dass der 1-Buten-Gehalt spürbaren Einfluss auf den Isoindex des erhaltenen $C_8$-Olefingemisches hat. Die Beispiele 2 bis 5 zeigen, dass das dem Stand der Technik bekannte Oligomerisierungsverfahren ungeeignet ist, für die Weichmacherherstellung brauchbare Di-Butene aus 1-Buten-armen $C_4$-Strömen zu produzieren.

Indes belegt der Vergleich der nicht erfindungsgemäßen Beispiele 2 bis 5 mit den erfindungsgemäßen Beispielen 6 bis 12, dass nach dem erfindungsgemäßen Verfahren $C_8$-Gemische mit geringem Isoindex von unter 1.05 selbst dann herstellbar sind, wenn der eingesetzte Eduktstrom nur geringste oder keine Anteile an 1-Buten enthält. Die dreistufigen Verfahren erzielen dabei einen Gesamtumsatz von annähernd 90 %. Die $C_8$-Selektivität liegt geringfügig über der des herkömmlichen Verfahrens.

Der erfindungsgemäß abgewandelte OCTOL-Prozess wurde also durch Begrenzung des Umsatzes in seinen einzelnen Oligomerisierungsschritten in besonderer Weise für die Verwertung von $C_4$-Einsatzgemischen mit einem geringen Gehalt an 1-Buten eingerichtet und liefert dennoch ein $C_8$-Produktgemisch mit einem für die Herstellung von Weichmacheralkoholen geeignetem Isoindex.

**Bezugszeichenliste**

| | |
|---|---|
| 1 | Eduktgemisch |
| 2 | Reaktionszone |
| 3 | Oligomerisat |
| 4 | Destillationskolonne |
| 5 | Kopfstrom |
| 6 | Sumpfstrom |
| 7 | Rückführstrom |
| 8 | Transferstrom |
| 9 | Bilanzgrenze |
| 10 | Gesamtprozess (zweitstufig) |
| 101 | erste Schrittsequenz |
| 102 | zweite Schrittsequenz |
| 11 | Eduktgemisch |
| 12 | erste Reaktionszone |
| 13 | erstes Oligomerisat |
| 14 | erste Destillationskolonne |
| 15 | erster Kopfstrom |
| 16 | erster Sumpfstrom |
| 17 | erster Rückführstrom |
| 18 | erster Transferstrom |
| 21 | für die zweite Stufe bereitgestelltes Kohlenwasserstoffgemisch |
| 22 | zweite Reaktionszone |
| 23 | zweites Oligomerisat |
| 24 | zweite Destillationskolonne |
| 25 | zweiter Kopfstrom |
| 26 | zweiter Sumpfstrom |
| 27 | zweiter Rückführstrom |
| 28 | zweiter Transferstrom |
| 29 | Fraktionierung |
| 30 | Verwertung |
| 32 | dritte Reaktionszone |
| 33 | drittes Oligomerisat |
| 34 | dritte Destillationskolonne |
| 36 | vereinigte Sumpfströme / dritter Sumpfstrom |
| 37 | $C_8$-Kolonne |
| 38 | $C_8$-Produktgemisch |
| 39 | Sumpfprodukt der $C_8$-Kolonne |
| 40 | $C_{12}$-Kolonne |

41    $C_{12}$-Produktgemisch

42    $C_{12+}$-Produktgemisch

**Patentansprüche**

1. Verfahren zur Herstellung von $C_8$-Olefinen und von $C_{12}$-Olefinen durch Oligomerisierung von Butenen, umfassend die folgende Schrittsequenz:

   a) Bereitstellen eines Kohlenwasserstoffgemisches enthaltend zumindest ein 2-Buten sowie mindestens ein weiteres, von diesem 2-Buten verschiedenes lineares Buten;
   b) Oligomerisieren eines Teils der in dem Kohlenwasserstoffgemisch enthaltenden Butene zu $C_8$-Olefinen und zu $C_{12}$-Olefinen und gegebenenfalls zu $C_{12+}$-Olefinen durch Inkontaktbringen des Kohlenwasserstoffgemisches mit einem in einer Reaktionszone angeordneten Oligomerisierungskatalysator bei einer in der Reaktionszone herrschenden Reaktionstemperatur unter Erhalt eines die hergestellten Oligomere und die nicht umgesetzten Butene enthaltenden Oligomerisats;
   c) Abtrennen der nicht umgesetzten Butene aus dem Oligomerisat;
   d) optional Rückführen eines Teils der abgetrennten, nicht umgesetzten Butene in die vorhergehende Oligomerisierung;

   wobei die Schrittsequenz mindestens einmal einschließlich des Schrittes "Rückführen" durchgeführt wird,
   **dadurch gekennzeichnet,**
   **dass** bei der erstmalig durchgeführten Schrittsequenz die auf die Konzentration an linearen Butenen bezogene Konzentration an 1-Buten in dem bereitgestellten Kohlenwasserstoffgemisch kleiner oder gleich der sich aus der Reaktionstemperatur der erstmalig durchgeführten Schrittsequenz ergebenen Gleichgewichtskonzentration an 1-Buten innerhalb der Fraktion der im erstmalig bereitgestellten Kohlenwasserstoffgemisch enthaltenden linearen Butene ist,
   **dass** die hergestellten $C_8$-Olefine als ein $C_8$-Produktgemisch erhalten werden, dessen Isoindex kleiner ist als 1.2, und **dass** bei der erstmalig durchgeführten Schrittsequenz der unmittelbar über die erste Reaktionszone bilanzierte Umsatz von Butenen begrenzt wird auf einen ersten Grenzwert zwischen 5 und 40 Gew.-%, welcher bevorzugt zwischen 10 und 38 Gew.-% und ganz besonders bevorzugt zwischen 10 und 36 Gew.-% liegt.

2. Verfahren nach Anspruch 1, wobei die Schrittsequenz "Bereitstellen, Oligomerisieren, Abtrennen und Rückführen" mindestens zweimal hintereinander durchgeführt wird,
   **dadurch gekennzeichnet,**
   **dass** bei der zweiten durchgeführten Schrittsequenz der unmittelbar an der zweiten Reaktionszone bilanzierte Umsatz von Butenen begrenzt wird auf einen zweiten Grenzwert zwischen 5 und 50 Gew.- %, welcher bevorzugt zwischen 10 und 40 Gew.-% liegt.

3. Verfahren nach Anspruch 2, wobei die Schrittsequenz "Bereitstellen, Oligomerisieren, Abtrennen und Rückführen" mindestens dreimal hintereinander durchgeführt wird,
   **dadurch gekennzeichnet,**
   **dass** bei der dritten durchgeführten Schrittsequenz der unmittelbar an der dritten Reaktionszone bilanzierte Umsatz von Butenen begrenzt wird auf einen dritten Grenzwert zwischen 5 und 65 Gew.-%, welcher bevorzugt zwischen 20 und 60 Gew.-% liegt.

4. Verfahren nach Anspruch 3, wobei die Schrittsequenz "Bereitstellen, Oligomerisieren, Abtrennen und Rückführen" mindestens viermal hintereinander durchgeführt wird,
   **dadurch gekennzeichnet,**
   **dass** bei der vierten durchgeführten Schrittsequenz der unmittelbar an der vierten Reaktionszone bilanzierte Umsatz von Butenen begrenzt wird auf einen vierten Grenzwert zwischen 5 und 80 Gew.-%, welcher bevorzugt zwischen 20 und 70 Gew.-% liegt.

5. Verfahren nach Anspruch 4, wobei die Schrittsequenz "Bereitstellen, Oligomerisieren, Abtrennen und Rückführen" mindestens fünfmal hintereinander durchgeführt wird,
   **dadurch gekennzeichnet,**
   **dass** bei der fünften durchgeführten Schrittsequenz der unmittelbar an der fünften Reaktionszone bilanzierte Umsatz von Butenen begrenzt wird auf einen fünften Grenzwert zwischen 5 und 95 Gew.-%, welcher bevorzugt zwischen 20 und 80 Gew.-% liegt.

6. Verfahren nach einem der vorhergehenden Ansprüche,
   **dadurch gekennzeichnet,**
   **dass** der nach Durchführung aller Schrittsequenzen erzielte Gesamtumsatz von Butenen zwischen 5 und 100 Gew.-% liegt, insbesondere, dass er zwischen 20 und 100 Gew.-% liegt und ganz besonders bevorzugt, dass er zwischen 30 und 95 Gew.-% liegt.

7. Verfahren nach einem der vorhergehenden Ansprüche,
   **dadurch gekennzeichnet,**
   **dass** die Begrenzung des Umsatzes in den jeweiligen Oligomerisierung dadurch erfolgt, dass die Reaktionstemperatur der jeweiligen Oligomerisierung insbesondere durch Einsatz eines externen Kühlmediums begrenzt wird auf eine Höchsttemperatur zwischen 40°C und 140°C, bevorzugt zwischen 45°C und 120°C und ganz besonders bevorzugt zwischen 50°C und 100°C.

8. Verfahren nach einem der vorhergehenden Ansprüche,
   **dadurch gekennzeichnet,**
   **dass** es sich bei den hergestellten Oligomeren zu mehr als 80 Gew.-% um $C_8$-Olefine handelt, insbesondere um mehr als 82 Gew.-% und ganz besonders bevorzugt um mehr als 84 Gew.-%.

9. Verfahren nach einem der vorhergehenden Ansprüche,
   **dadurch gekennzeichnet,**
   **dass** sich die hergestellten $C_8$-Olefine wie folgt zu 100 Gew.-% ergänzend zusammensetzen:

   n-Octene: 10 bis 25 Gew.-%, bevorzugt 12 bis 20 Gew.-% und besonders bevorzugt 14 bis 20 Gew.-%;
   Metylheptene: 50 bis 80 Gew.-%, bevorzugt 55 bis 75 Gew.-% und besonders bevorzugt 60 bis 70 Gew.-%;
   Dimethylhexene: 10 bis 30 Gew.-%, bevorzugt 10 bis 25 Gew.-% und besonders bevorzugt 10 bis 20 Gew.-%.

10. Verfahren nach einem der vorhergehenden Ansprüche,
    **dadurch gekennzeichnet,**
    **dass** das $C_8$-Produktgemisch einen Isoindex kleiner 1.1 und besonders bevorzugt von kleiner als 1.05 aufweist.

11. Verfahren nach einem der vorhergehenden Ansprüche,
    **dadurch gekennzeichnet,**
    **dass** es sich es sich bei dem erstmalig bereitgestellten Kohlenwasserstoffgemisch um ein Eduktgemisch mit der folgenden, sich zu 100 Gew.-% ergänzenden Zusammensetzung handelt:

    | | |
    |---|---|
    | 1-Buten: | weniger als 10 Gew.-%, bevorzugt weniger als 5 Gew.-% und besonders bevorzugt weniger als 2 Gew.-%; |
    | 2-Butene: | 20 bis 90 Gew.-% |
    | Isobuten: | weniger als 5 Gew.-%, bevorzugt weniger als 1 Gew.-% |
    | n-Butan: | weniger als 80 Gew.-% |
    | Isobutan: | weniger als 80 Gew.-% |
    | Sonstige: | weniger als 5 Gew.-%, bevorzugt weniger als 2 Gew.-% |

12. Verfahren nach einem der vorhergehenden Ansprüche,
    **dadurch gekennzeichnet,**
    **dass** es in einer Anlage durchgeführt wird, innerhalb welcher jeder durchgeführten Schrittsequenz genau eine Reaktionszone zugeordnet ist.

13. Verfahren nach einem der vorhergehenden Ansprüche,
    **dadurch gekennzeichnet,**
    **dass** in jeder durchlaufenen Schrittsequenz mit Ausnahme der letzten Schrittsequenz der nicht rückgeführte Teil der abgetrennten, nicht umgesetzten Butene als Kohlenwasserstoffgemisch für die darauffolgende Schrittsequenz bereitgestellt wird.

**14.** Verfahren nach einem der vorhergehenden Ansprüche,
**dadurch gekennzeichnet,**
**dass** der in der letzten Schrittsequenz nicht rückgeführte Teil der abgetrennten, nicht umgesetzten Butene verwertet wird, wobei die Verwertung ausgewählt ist aus den folgenden Verwertungsmöglichkeiten:

    a) Vollhydrierung unter Erhalt eines Butangemisches;
    b) Oxidative oder nichtoxidative Dehydrierung zu Butadien;
    c) Hydroformylierung zu Pentanalen;
    d) Oxidation insbesondere zu Maleinsäureanhydrid
    e) Metathese;
    f) Hydratisierung zu Butanolen,
    g) Alkylierung;
    h) Isomerisierung;
    i) Addition von Alkoholen zu Ethern;
    j) Carbonylierung;
    k) Cracken in einem Steamcracker oder einem fluidkatalytischen Cracker unter Erhalt von Kohlenwasserstoffen mit weniger als vier Kohlenstoffatomen;
    l) Verbrennen unter Erhalt thermischer Energie.

**15.** Verfahren nach einem der vorhergehenden Ansprüche,
**dadurch gekennzeichnet,**
**dass** die in den einzelnen Schrittsequenzen erhaltenen Oligomere vereinigt und anschließend aufgetrennt werden in das $C_8$-Produktgemisch, in ein $C_{12}$-Produktgemisch und in ein $C_{12+}$-Produktgemisch.

**Claims**

**1.** Process for preparing $C_8$ olefins and $C_{12}$ olefins by oligomerization of butenes, comprising the following step sequence:

    a) providing of a hydrocarbon mixture comprising at least one 2-butene and also at least one further linear butene other than this 2-butene;
    b) oligomerizing of a part of the butenes comprised in the hydrocarbon mixture, to give $C_8$ olefins and to give $C_{12}$ olefins and optionally to give $C_{12+}$ olefins, by contacting of the hydrocarbon mixture with an oligomerization catalyst arranged in a reaction zone, at a reaction temperature prevailing in the reaction zone, to give an oligomerizate comprising the prepared oligomers and the unreacted butenes;
    c) removing of the unreacted butenes from the oligomerizate;
    d) optionally, recycling of a part of the removed unreacted butenes to the preceding oligomerization;

the step sequence being carried out at least once with inclusion of the "recycling" step,
**characterized**
**in that** in the step sequence carried out for the first time, the concentration of 1-butene in the provided hydrocarbon mixture, based on the concentration of linear butenes, is less than or equal to the equilibrium concentration of 1-butene, resulting from the reaction temperature of the step sequence carried out for the first time, within the fraction of the linear butenes comprised in the hydrocarbon mixture provided for the first time,
**in that** the $C_8$ olefins prepared are obtained as a $C_8$ product mixture whose Iso index is less than 1.2,
and **in that** in the step sequence carried out for the first time, the conversion of butenes, assessed directly over the first reaction zone, is limited to a first limit value of between 5 and 40 wt%, this value lying preferably between 10 and 38 wt% and very preferably between 10 and 36 wt%.

**2.** Process according to Claim 1, the "providing, oligomerizing, removing and recycling" step sequence being carried out at least two times one after another,
**characterized**
**in that** in the second step sequence carried out, the conversion of butenes, assessed directly at the second reaction zone, is limited to a second limiting value of between 5 and 50 wt%, said value lying preferably between 10 and 40 wt%.

**3.** Process according to Claim 2, the "providing, oligomerizing, removing and recycling" step sequence being carried out at least three times one after another,

**characterized**

**in that** in the third step sequence carried out, the conversion of butenes, assessed directly at the third reaction zone, is limited to a third limiting value of between 5 and 65 wt%, said value lying preferably between 20 and 60 wt%.

4.  Process according to Claim 3, the "providing, oligomerizing, removing and recycling" step sequence being carried out at least four times one after another,
    **characterized**
    **in that** in the fourth step sequence carried out, the conversion of butenes, assessed directly at the fourth reaction zone, is limited to a fourth limiting value of between 5 and 80 wt%, said value lying preferably between 20 and 70 wt%.

5.  Process according to Claim 4, the "providing, oligomerizing, removing and recycling" step sequence being carried out at least five times one after another,
    **characterized**
    **in that** in the fifth step sequence carried out, the conversion of butenes, assessed directly at the fifth reaction zone, is limited to a fifth limiting value of between 5 and 95 wt%, said value lying preferably between 20 and 80 wt%.

6.  Process according to any of the preceding claims,
    **characterized**
    **in that** the overall conversion of butenes achieved after all of the step sequences have been carried out is between 5 and 100 wt%, more particularly in that it lies between 20 and 100 wt% and very preferably in that it lies between 30 and 95 wt%.

7.  Process according to any of the preceding claims,
    **characterized**
    **in that** the limiting of the conversion in the respective oligomerization is accomplished by limiting the reaction temperature of the respective oligomerization, more particularly through use of an external cooling medium, to a maximum temperature of between 40°C and 140°C, preferably between 45°C and 120°C and very preferably between 50°C and 100°C.

8.  Process according to any of the preceding claims,
    **characterized**
    **in that** more than 80 wt% of the oligomers prepared are $C_8$ olefins, more particularly more than 82 wt% and very preferably more than 84 wt%.

9.  Process according to any of the preceding claims,
    **characterized**
    **in that** the composition of the $C_8$ olefins prepared is as follows, adding up to 100 wt%:

|  |  |
|---|---|
| n-octenes: | 10 to 25 wt%, preferably 12 to 20 wt% and more preferably 14 to 20 wt%; |
| methylheptenes: | 50 to 80 wt%, preferably 55 to 75 wt% and more preferably 60 to 70 wt%; |
| dimethylhexenes: | 10 to 30 wt%, preferably 10 to 25 wt% and more preferably 10 to 20 wt%. |

10. Process according to any of the preceding claims,
    **characterized**
    **in that** the $C_8$ product mixture has an Iso index of less than 1.1 and more preferably of less than 1.05.

11. Process according to any of the preceding claims,
    **characterized**
    **in that** the hydrocarbon mixture provided for the first time is a reactant mixture having the following composition, which adds up to 100 wt%:

1-butene: less than 10 wt%, preferably less than 5 wt% and more preferably less than 2 wt%
2-butenes: 20 to 90 wt%

isobutene: less than 5 wt%, preferably less than 1 wt%
n-butane: less than 80 wt%
isobutane: less than 80 wt%
others: less than 5 wt%, preferably less than 2 wt%

**12.** Process according to any of the preceding claims,
**characterized**
**in that** it is carried out in a plant within which exactly one reaction zone is assigned to each step sequence carried out.

**13.** Process according to any of the preceding claims,
**characterized**
**in that** in each traversed step sequence apart from the last step sequence, the non-recycled part of the removed unreacted butenes is provided as a hydrocarbon mixture for the subsequent step sequence.

**14.** Process according to any of the preceding claims,
**characterized**
**in that** the part of the removed unreacted butenes not recycled in the last step sequence is productively utilized, this utilization being selected from the following utilization possibilities:

  a) total hydrogenation to give a butane mixture;
  b) oxidative or non-oxidative dehydrogenation to butadiene;
  c) hydroformylation to pentanals;
  d) oxidation in particular to maleic anhydride;
  e) metathesis;
  f) hydration to butanols;
  g) alkylation;
  h) isomerization;
  i) addition reaction of alcohols to ethers;
  j) carbonylation;
  k) cracking in a steamcracker or in a fluid-catalytic cracker to give hydrocarbons having less than four carbon atoms;
  l) combustion to give thermal energy.

**15.** Process according to any of the preceding claims,
**characterized**
**in that** the oligomers obtained in the individual step sequences are combined and then separated into the $C_8$ product mixture, into a $C_{12}$ product mixture and into a $C_{12+}$ product mixture.

**Revendications**

**1.** Procédé de fabrication d'oléfines en $C_8$ et d'oléfines en $C_{12}$ par oligomérisation de butènes, comprenant la séquence d'étapes suivante :

  a) la préparation d'un mélange d'hydrocarbures contenant au moins un 2-butène et au moins un autre butène linéaire différent de ce 2-butène ;
  b) l'oligomérisation d'une partie des butènes contenus dans le mélange d'hydrocarbures en oléfines en $C_8$ et en oléfines en $C_{12}$ et éventuellement en oléfines en $C_{12+}$ par mise en contact du mélange d'hydrocarbures avec un catalyseur d'oligomérisation agencé dans une zone de réaction à une température de réaction régnant dans la zone de réaction afin d'obtenir un produit oligomérisé contenant les oligomères fabriqués et les butènes non réagis ;
  c) la séparation des butènes non réagis du produit oligomérisé ;
  d) éventuellement le recyclage d'une partie des butènes non réagis séparés dans l'oligomérisation précédente ; la séquence d'étapes étant réalisée au moins à une reprise, y compris l'étape « recyclage »,

  **caractérisé en ce que** lors de la séquence d'étapes réalisée pour la première fois, la concentration en 1-butène relative à la concentration en butènes linéaires dans le mélange d'hydrocarbures préparé est inférieure ou égale à la concentration à l'équilibre en 1-butène dans la fraction des butènes linéaires contenus dans le mélange d'hydrocarbures préparé pour la première fois découlant de la température de réaction de la séquence d'étapes réalisée pour la première fois,

les oléfines en $C_8$ fabriquées sont obtenues sous la forme d'un mélange de produits en $C_8$, dont l'indice iso est inférieur à 1,2,

et lors de la séquence d'étapes réalisée pour la première fois, la conversion des butènes établie directement sur la première zone de réaction est limitée à une première valeur limite comprise entre 5 et 40 % en poids, qui est de préférence comprise entre 10 et 38 % en poids et de manière tout particulièrement préférée comprise entre 10 et 36 % en poids.

2. Procédé selon la revendication 1, dans lequel la séquence d'étapes « préparation, oligomérisation, séparation et recyclage » est réalisée successivement au moins à deux reprises, **caractérisé en ce que** lors de la deuxième séquence d'étapes réalisée, la conversion des butènes établie directement sur la deuxième zone de réaction est limitée à une deuxième valeur limite comprise entre 5 et 50 % en poids, qui est de préférence comprise entre 10 et 40 % en poids.

3. Procédé selon la revendication 2, dans lequel la séquence d'étapes « préparation, oligomérisation, séparation et recyclage » est réalisée successivement au moins à trois reprises, **caractérisé en ce que** lors de la troisième séquence d'étapes réalisée, la conversion des butènes établie directement sur la troisième zone de réaction est limitée à une troisième valeur limite comprise entre 5 et 65 % en poids, qui est de préférence comprise entre 20 et 60 % en poids.

4. Procédé selon la revendication 3, dans lequel la séquence d'étapes « préparation, oligomérisation, séparation et recyclage » est réalisée successivement au moins à quatre reprises, **caractérisé en ce que** lors de la quatrième séquence d'étapes réalisée, la conversion des butènes établie directement sur la quatrième zone de réaction est limitée à une quatrième valeur limite comprise entre 5 et 80 % en poids, qui est de préférence comprise entre 20 et 70 % en poids.

5. Procédé selon la revendication 4, dans lequel la séquence d'étapes « préparation, oligomérisation, séparation et recyclage » est réalisée successivement au moins à cinq reprises, **caractérisé en ce que** lors de la cinquième séquence d'étapes réalisée, la conversion des butènes établie directement sur la cinquième zone de réaction est limitée à une cinquième valeur limite comprise entre 5 et 95 % en poids, qui est de préférence comprise entre 20 et 80 % en poids.

6. Procédé selon l'une quelconque des revendications précédentes, **caractérisé en ce que** la conversion totale des butènes obtenue après la réalisation de toutes les séquences d'étapes est comprise entre 5 et 100 % en poids, notamment entre 20 et 100 % en poids et de manière tout particulièrement préférée entre 30 et 95 % en poids.

7. Procédé selon l'une quelconque des revendications précédentes, **caractérisé en ce que** la limitation de la conversion dans l'oligomérisation respective a lieu par limitation de la température de réaction de l'oligomérisation respective, notamment en utilisant un agent réfrigérant externe, à une température maximale comprise entre 40 °C et 140 °C, de préférence entre 45 °C et 120 °C et de manière tout particulièrement préférée entre 50 °C et 100 °C.

8. Procédé selon l'une quelconque des revendications précédentes, **caractérisé en ce que** les oligomères fabriqués sont à hauteur de plus de 80 % en poids des oléfines en $C_8$, notamment à hauteur de plus de 82 % en poids et de manière tout particulièrement préférée à hauteur de plus de 84 % en poids.

9. Procédé selon l'une quelconque des revendications précédentes, **caractérisé en ce que** les oléfines en $C_8$ fabriquées ont la composition suivante, dont la somme est de 100 % en poids :

n-octènes : 10 à 25 % en poids, de préférence 12 à 20 % en poids et de manière particulièrement préférée 14 à 20 % en poids ;
méthylheptènes : 50 à 80 % en poids, de préférence 55 à 75 % en poids et de manière particulièrement préférée 60 à 70 % en poids ;
diméthylhexènes : 10 à 30 % en poids, de préférence 10 à 25 % en poids et de manière particulièrement préférée 10 à 20 % en poids.

**10.** Procédé selon l'une quelconque des revendications précédentes, **caractérisé en ce que** le mélange de produits en C$_8$ présente un indice iso inférieur à 1, et de manière particulièrement préférée inférieur à 1,05.

**11.** Procédé selon l'une quelconque des revendications précédentes, **caractérisé en ce que** le mélange d'hydrocarbures préparé pour la première fois consiste en un mélange de réactifs ayant la composition suivante, dont la somme est de 100 % en poids :

1-butène : moins de 10 % en poids, de préférence moins de 5 % en poids et de manière particulièrement préférée moins de 2 % en poids ;
2-butènes : 20 à 90 % en poids,
isobutène : moins de 5 % en poids, de préférence moins de 1 % en poids,
n-butane : moins de 80 % en poids,
isobutane : moins de 80 % en poids,
autres : moins de 5 % en poids, de préférence moins de 2 % en poids.

**12.** Procédé selon l'une quelconque des revendications précédentes, **caractérisé en ce qu'**il est réalisé dans une unité dans laquelle chaque séquence d'étapes réalisée correspond exactement à une zone de réaction.

**13.** Procédé selon l'une quelconque des revendications précédentes, **caractérisé en ce que** dans chaque séquence d'étapes traversée, à l'exception de la dernière séquence d'étapes, la partie non recyclée des butènes non réagis séparés est préparée en tant que mélange d'hydrocarbures pour la séquence d'étapes suivante.

**14.** Procédé selon l'une quelconque des revendications précédentes, **caractérisé en ce que** la partie non recyclée des butènes non réagis séparés dans la dernière séquence d'étapes est exploitée, l'exploitation étant choisie parmi les possibilités d'exploitation suivantes :

a) l'hydrogénation totale pour obtenir un mélange de butanes ;
b) la déshydrogénation oxydative ou non oxydative en butadiène ;
c) l'hydroformylation en pentanals ;
d) l'oxydation, notamment en anhydride de l'acide maléique ;
e) la métathèse ;
f) l'hydratation en butanols ;
g) l'alkylation ;
h) l'isomérisation ;
i) l'addition d'alcools en éthers ;
j) la carbonylation ;
k) le craquage dans un vapocraqueur ou un craqueur catalytique fluide pour obtenir des hydrocarbures contenant moins de quatre atomes de carbone ;
l) la combustion pour obtenir de l'énergie thermique.

**15.** Procédé selon l'une quelconque des revendications précédentes, **caractérisé en ce que** les oligomères obtenus dans les séquences d'étapes individuelles sont réunis, puis séparés en le mélange de produits en C$_8$, en un mélange de produits en C$_{12}$ et en un mélange de produits en C$_{12}$+.

Fig. 1

Fig. 2

$C_4$

11        12        14        22        24        32        34        $C_4$

$C_8, C_{12,} C_{12+}$

38        $C_8$

41        $C_{12}$

36        37

40

39    $C_{12,} C_{12+}$    42        $C_{12+}$

Fig. 3

Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008007081 A1
- EP 1029839 A1
- WO 9925668 A
- DE 10015002 A1
- WO 9925668 A1
- DE 4339713 A1
- WO 200137989 A2
- WO 2011000697 A1


**In der Beschreibung aufgeführte Nicht-Patentliteratur**

- **F. GEILEN ; G. STOCHNIOL ; S. PEITZ ; E. SCHULTE-KOERNE.** Butenes. Ullmann's Encyclopedia of Industrial Chemistry. 2013
- **B. SCHOLZ.** The HÜLS OCTOL Process: Heterogeneously catalyzed dimerization of n-butenes and other olefins. *DGMK-Tagung in Karlsruhe,* April 1989, 21, , 22
- **R.H. FRIEDLANDER ; D.J. WARD ; F. OBENAUS ; F. NIERLICH ; J. NEUMEISTER.** Make plasticizer olefins via n-butene dimerization. *Hydrocarbon Processing,* Februar 1986, 31-33
- **F. NIERLICH.** Oligomerize for better gasoline. *Hydrocarbon Processing,* Februar 1992, 45-46
- **F. NIERLICH ; ERDÖL ; ERDGAS ; KOHLE. JG.** *Integrated tert. butyl Alcohol / Di-n-butenes Production from FCC C4's,* 1987, vol. 103, 486-489